# EUROPEAN PATENT SPECIFICATION

(11) **EP 0 500 947 B1**
(45) Date of publication and mention of the grant of the patent: **06.05.1998**
(21) Application number: 91914761.1
(22) Date of filing: 22.08.1991
(51) Int. Cl.: G01N 33/579

(54) **ASSAYING AGENT FOR ENDOTOXIN**
TESTAGENS FÜR ENDOTOXIN
AGENT DE DETECTION DE L'ENDOTOXINE

(30) Priority: 22.08.1990 JP 218954/90
(43) Date of publication of application: 02.09.1992
(73) Proprietor: SEIKAGAKU KOGYO KABUSHIKI KAISHA (SEIKAGAKU CORPORATION), Tokyo 103 (JP)
(72) Inventor: TANAKA, Shigenori, 3-15, Ogawanishimachi 5-chome, Tokyo 187 (JP); TAMURA, Hiroshi, 2-408 Green-Town, Tokyo 207 (JP)
(74) Representative: Hansen, Bernd, Dr. Dipl.-Chem.
(86) International application number: PCT/JP91/01118
(87) International publication number: WO 92/03736

(56) References cited:
- EP-A- 0 291 856
- JP-A- 1 219 562
- JP-A- 1 235 852
- JP-A- 1 503 808
- JP-A-58 013 516
- JP-A-59 027 828
- JP-A-60 125 565
- US-A- 4 414 336

## Description

### FIELD OF THE INVENTION

This invention relates to a reagent for assaying an endotoxin with the use of limulus amebocyte lysate.

### BACKGROUND OF THE INVENTION

There has been known a method for assaying an endotoxin with the use of limulus amebocyte lysate (hereinafter referred to simply as lysate) by taking advantage of a fact that said lysate coagulates with a trace amount of an endotoxin. Subsequent biochemical studies have revealed that this coagulation is caused by stepwise activation of several coagulation factors [cf. Takanori Nakamura et al., Japanese Journal of Bacteriology, 38, 781 - 803 (1983)].

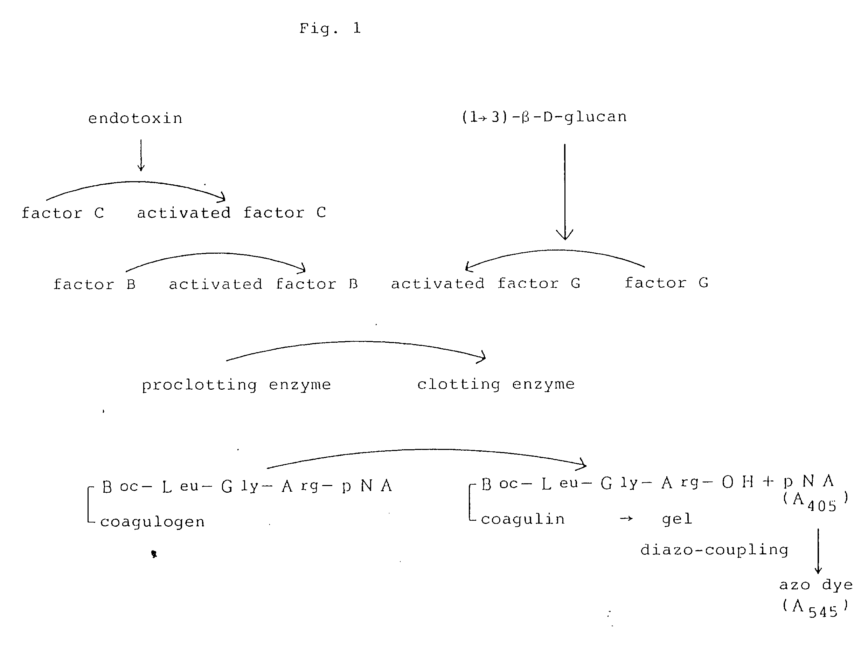

When an endotoxin is added to the lysate, as shown in Fig. 1, factor C (an endotoxin-sensitive factor, molecular weight: 123,000) is activated. Then, the activated factor C definitively hydrolyzes and activates factor B (molecular weight: 64,000). The activated factor B thus formed activates proclotting enzyme (molecular weight: 54,000) and thus convert the same into clotting enzyme. The clotting enzyme definitively hydrolyzes specific sites, i.e., Arg¹⁸-Thr¹⁹ and Arg⁴⁶-Gly⁴⁷, of coagulogen (a coagulation protein, molecular weight: 19,723) and thus liberates peptide C to thereby convert the coagulogen into coagulin, which causes coagulation (gelation). Iwanaga et al. [Haemostasis, 7, 183 - 199 (1978)] further proposed a highly quantitative assay method wherein the lysate is combined with a synthetic peptide having an amino acid sequence in common with the above-mentioned hydrolysis sites of coagulogen, namely a chromogenic substrate Boc-Leu-Gly-Arg-p-nitroanilide (pNA) or a fluorogenic substrate Boc-Leu-Gly-Arg-4-methylcoumaryl-7-amide.

This assay method depends on a series of reactions involving a cascade mechanism wherein an endotoxin acts as a trigger so as to successively activate coagulation factors all of which are serine protease precursors and thus the coagulin is formed finally.

When (1 → 3)-β-D-glucan is added to the lysate, factor G shown in Fig. 1 is activated and the activated factor G thus formed converts the proclotting enzyme into the clotting enzyme. Next, the clotting enzyme converts the coagulogen into the coagulin, similar to the case of the endotoxin, to thereby form a gel and hydrolyzes a synthetic substrate [cf. Morita et al., FEBS Lett., 129, 318 - 321 (1981)].

Known examples of substances reacting with factor G include (1 → 3)-β-D-glucan, krestin, lentinan and substances contained in rinses from cellulosic hemodialyzer and blood contacted therewith. It has been confirmed that none of these substances would show any positive reaction in a rabbit pyrogenic test.

Endotoxin is also known as cell wall components of gram-negative bacteria. Thus, the presence of gram-negative bacteria in vivo can be detected by determining endotoxin in blood. Accordingly, it has been urgently required in the field of clinical diagnosis to establish a method of assaying an endotoxin at a high sensitivity and a high reproducibility without being affected by (1 3)-β-D-glucan.

Obayashi et al. reported a method of assaying an endotoxin by using factor C-system in the lysate [cf. Clin. Chim. Acta, 149, 55 - 65 (1985)]. However, this method requires a highly complicated procedure since it comprises fractionating the lysate by gel filtration or affinity chromatography with the use of a heparin or dextran sulfate-immobilized carrier, eliminating factor G sensitive to (1 → 3)-β-D-glucan and thus reconstituting the lysate with factors C and B and the proclotting enzyme.

EP 291 856 describes a 2-component kit comprising an antibody raised against the component of horseshoe crab amoebocytes lysate and a component of horseshoe crab amoebocytes lysate, as well as a method for determining the presence of an endotoxin in which a sample is incubated with a component of horseshoe crab amoebocytes lysate and the sample-component mixture is then incubated with an antibody raised against the component of said lysate.

### DESCRIPTION OF THE INVENTION

The present invention relates to a reagent for assaying an endotoxin through a reaction of factors C and B in lysate by using an antibody to the (1 → 3)-β-D-glucan-sensitive factor so as to eliminate any effects of factor G sensitive to (1 → 3)-β-D-glucan.

Namely, the reagent for assaying an endotoxin of the present invention includes:
a reagent for assaying an endotoxin which contains limulus amoebocyte lysate, an antibody to (1→3)-β-D-glucan sensitive factor and a synthetic peptide substrate having an amino acid sequence comprising a hydrolysis site of coagulogen.

As described above, the (1 → 3)-β-D-glucan-sensitive factor is factor G activated by (1 → 3)-β-D-glucan. The effects of factor G contained in the lysate should be eliminated to specifically assay an endotoxin by using the lysate without being affected by (1 → 3)-β-D-glucan. In order to solve this problem, an antibody to factor G is used together with the lysate or a lysate substantially free from any factor G obtained by using anti-factor G antibody-immobilized carrier.

The lysate used in the present invention may be obtained by collecting the hemolymph of horseshoe crab such as Limulus polyphemus in U.S.A., Tachypleus gigas in Thailand and Malaysia, Tachypleus tridentatus in Japan and China, Carcinoscorpius rotundicauda in Thailand and Malaysia, grinding the blood cells and separating the component (lysate). It is preferable that the lysate is stored at -40 °C or below in small portions and thawed and dissolved upon use.

In order to produce an antibody to factor G from the lysate thus obtained, it is first required to purify factor G which serves as an antigen. Factor G may be purified by contacting the lysate with an appropriate carrier such as agarose, Sepharose (trade name of a product commercially available from Pharmacia), crosslinked derivatives thereof, on which dextran sulfate or heparin is immobilized, and then collecting fractions containing factor G. These processes may be conducted by, for example, contacting the above-described immobilized carrier with the lysate in a solution or by utilizing a column chromatography.

An antibody to (1 → 3)-β-D-glucan-sensitive factor is produced by using as an antigen the purified factor G sensitive to (1 → 3)-β-D-glucan or the factor G fraction free from factors C and B. Thus, polyclonal and monoclonal antibodies to these antigens are obtained.

The polyclonal antibody used in the present invention may be produced by administering the above-mentioned antigen to an animal to be immunized (for example, rabbit, goat). It is preferable to further purify the antibody thus obtained. When the antigen is administered to the animal, it is recommended to use an adjuvant since the antibody-producing cells can be activated thereby.

The monoclonal antibody used in the present invention may be produced by, for example, the following method. Namely, the above-mentioned antigen is intraperitoneally administered to a rat or a mouse and then the spleen of the animal is taken out. Cells collected from this spleen are fused with myeloma cells which are cells of tumor cell line to obtain hybridomas. After continuously proliferating these hybridomas in vitro, a cell line capable of continuously producing a specific antibody to the above-mentioned antigen is selected. Then, the selected cells are incubated either in vitro or in vivo (for example, in the abdominal cavity of a mouse) to produce a large amount of the monoclonal antibody. In addition to the above-mentioned spleen cells, lymph node cells and lymphocytes in peripheral blood may be used in the cell fusion. Preferable as the myeloma cells are those derived from the same species as compared to those derived from a different species, since hybridomas which stably produces the antibody can be obtained.

The polyclonal and monoclonal antibody thus obtained may be purified by salting out with a neutral salt such as sodium sulfate and ammonium sulfate, a cold alcohol precipitation or selective fractional precipitation using polyethylene glycol or utilizing an isoelectric point, electrophoresis, adsorption and desorption using an ion exchanger such as DEAE- or CM-derivatives, or adsorbents such as protein A or hydroxyapatite, gel filtration and ultracentrifugation.

In the above method of assaying an endotoxin, the antibody may be combined with a solution of the lysate and endotoxin by the following methods: a method comprising dissolving a freeze-dried product of a mixture of the lysate and a synthetic substrate in an appropriate buffer solution and adding thereto a solution of the antibody; a method comprising freeze-drying a mixture of the lysate, a synthetic substrate and the required amount of a solution of the antibody and dissolving the freeze-dried preparation in distilled water or an appropriate buffer solution; and a method comprising adding the required amount of a solution of the antibody to a sample.

Examples of the biological samples for assaying an endotoxin by the method of the present invention include blood, plasma and serum as well as endogenous and exogenous exudates and excretes such as cerebrospinal fluid, ascites, articular fluid, pleural effusion, milk and urine. When plasma is used as a sample, for example, it should be separated by adding an anticoagulant such as heparin, EDTA and citric acid.

An endotoxin may be assayed using the assay reagent of the present invention by the following methods: a method comprising adding a synthetic substrate such as the above-mentioned chromogenic or fluorogenic substrate to the reaction mixture and then determining the amidolytic activity of the clotting enzyme; a gelation method comprising examining gel formation due to coagulation with the naked eye; a turbidimetric method comprising measuring turbidity accompanying the coagulation by using an appropriate optical system; a turbidimetric kinetic assay comprising measuring the time required for achieving a specific turbidity by using an appropriate optical system; and a method of measuring a change in viscosity accompanying the coagulation in terms of a change in resonant frequency using a quartz chemical analyzer.

It is the first characteristic of the reagent for assaying an endotoxin of the present invention, which contains an antibody for factor G, that it shows an excellent ability to specifically bind to factor G and a neutralizing effect, even in a small amount. It is the second characteristic of the same that the antibody is free from any serine protease inhibitors such as antitrypsin and antithrombin III known as inhibitors of the limulus reaction and thus never deteriorates factor C activity.

### BRIEF DESCRIPTION OF THE DRAWINGS

Fig. 1 shows the cascade mechanism of limulus blood coagulation system.

Fig. 2 shows the reactivities of reagents A and D against (1 → 3)-β-D-glucan. Fig. 3 shows the reactivities of the reagents A and D against E. coli 0111:B4 endotoxin. Fig. 4 shows the reactivities of the reagent D against Salmonella enteritidis endotoxin diluted with water and a solution containing (1 → 3)-β-D-glucan.

Fig. 5 shows calibration curves of E. coli 0111:B4 endotoxin prepared in Examples 8 to 10.

Fig. 6 shows the results of the assay of endotoxin contained in plasma specimens in Example 8.

To further illustrate the present invention in greater detail, the following Examples will be given.

### BEST MODE FOR PRACTICING THE INVENTION

### Example 1

### Production of polyclonal antibody to factor G

One litter of limulus hemolymph was centrifuged at 4 °C at 1,500 rpm for 10 minutes. To approximately 50 g of the precipitate (amebocyte) thus obtained was added 250 ml of 0.02 M Tris-HCl buffer (pH 8.0). The mixture was homogeneously ground in a homogenizer (Polytoron R PT10 (trade mark), manufactured by Kinematica Co.) followed by extraction and centrifuged in a refrigerated centrifuge (Tomy Seiko RD-20III) at 10,000 rpm for 30 minutes. The resulting precipitate was further extracted with 150 ml portions of the above-mentioned buffer twice. Thus, 550 ml of the lysate was finally obtained.

The whole lysate thus obtained was applied to a dextran sulfate-immobilized Sepharose CL-6B column [5 x 23 cm, equilibrated with 0.02 M Tris-HCl buffer (pH 8.0) containing 0.05 M NaCl] and eluted with a 0.02 M Tris-HCl buffer (pH 8.0) containing 0.2 M NaCl. The factor G fraction thus eluted, which contains factor G as shown in Fig. 1, was determined for G factor activity by the method of Obayashi et al. as described below [Clin. Chim. Acta, 149, 55 - 65 (1985)]. A 50 ml portion of the fraction was concentrated to 10 ml under reduced pressure and 0.23 g of EDTA-4Na was added thereto to inhibit the activation of factor G.

To 1.0 ml of the concentrate was added the equivalent amount of Freund's complete adjuvant (trade name of a product commercially available from Iatron Co.). Then, a rabbit (JW, male, 2.5 kg) was sensitized by subcutaneously injecting 0.3 ml, 0.3 ml and 0.4 ml portions of the mixture respectively at the back, hip and the side of the abdomen. The sensitization was effected once in 2 weeks (5 times in all). After confirming an increase in the antibody titer by the double immunodiffusion, the cervical vein was incised and the whole blood was collected one week after the final sensitization. The blood was allowed to stand at room temperature for 1 hour and then at 4 °C overnight. After centrifuging at 2,000 rpm for 5 minutes, 52 ml of the serum thus obtained was inactivated by heating at 56 °C for 30 minutes. To a 50 ml portion of the serum was added 50 ml of a 34 % (w/v) Na₂SO₄ solution and the precipitate thus formed was centrifuged at 10,000 rpm for 30 minutes. The resulting precipitate was washed with a 17 % (w/v) Na₂SO₄ solution twice and the precipitate was dissolved in 50 ml of 0.1 M Tris-HCl buffer (pH 8.0). 7.5 g of solid Na₂SO₄ was dissolved in this solution under stirring. The precipitate thus formed was dissolved in the same Tris-HCl buffer as used above. Further, the precipitation procedure was repeated thrice by using an Na₂SO₄ solution at a concentration of 7.5 g/50 ml and the precipitate thus finally obtained was dissolved in the above-mentioned buffer solution. Subsequently, it was desalted by passing through a Cellulofine GH-20m (trade name of a product commercially available from Seikagaku Corporation) column (2.8 x 90 cm, eluted with 0.05 M NH₄HCO₃) equilibrated with 0.05 M NH₄HCO₃. The eluate was freeze-dried, to obtain an IgG fraction of rabbit anti-factor G fraction serum.

### [Assay of factor G activity]

To 0.1 ml of a 0.2 M Tris-HCl buffer (pH 8.0, containing 0.013 M MgCl₂) were added 0.03 ml of (1 → 3)-β-D-glucan (curdlan, trade name of a product commercially available from Wako Pure Chemicals Industries, Ltd.; 400 ng/ml), 0.05 ml of each fraction, 0.02 ml of 0.005 M N-tert-butoxycarbonyl(Boc)-Leu-Gly-Arg-p-nitroanilide (pNA) and 0.05 ml of proclotting enzyme and the mixture was allowed to react at 37 °C. After confirming coloration, the reaction was stopped by adding 0.8 ml of 0.6 M acetic acid. Then, the absorbance at 405 nm was measured.

### Example 2

### Production of polyclonal antibody to purified factor G

1.2 litter of limulus hemolymph was centrifuged at 4 °C at 1,500 rpm for 10 minutes. To approximately 53 g of the precipitate (amebocyte) thus obtained was added 250 ml of 0.02 M Tris-HCl buffer (pH 8.0, containing 0.001 M benzamidine and 0.001 M EDTA-4Na). The mixture was homogeneously ground, extracted and centrifuged at 10,000 rpm for 30 minutes in the same manner as in Example 1. The resulting precipitate was further extracted with 200 ml portions of the above-mentioned buffer twice. Thus, 640 ml of the lysate was finally obtained.

The whole lysate thus obtained was applied to a dextran sulfate-immobilized Sepharose CL-6B column [5 x 23.5 cm, equilibrated with 0.02 M Tris-HCl buffer (pH 8.0)] and eluted with a 0.02 M Tris-HCl buffer (pH 8.0) containing 0.2 M NaCl. The factor G fraction thus eluted was applied to a Columnlite (trade name of product commercially available from Seikagaku Corporation) column [3.0 x 29.6 cm, equilibrated with 0.02 M Tris-HCl buffer (pH 8.0)] and washed with 800 ml portions of a 0.02 M Tris-HCl buffer (pH 8.0) and 0.5 M NH₄HCO₃. After eluting with 2 M NH₄HCO₃, purified factor G was obtained.

50 ml of the above-mentioned purified factor G solution was concentrated to 10 ml and then 0.23 g of EDTA-4Na was added thereto so as to inhibit the activation of factor G.

To 1.0 ml of the concentrate was added the equivalent amount of Freund's complete adjuvant. Then, a rabbit (JW, male, 2.5 kg) was sensitized by subcutaneously injecting 0.3 ml, 0.3 ml and 0.4 ml portions of the mixture respectively at the back, hip and the side of the abdomen. The sensitization was effected once in 2 weeks (5 times in all). After confirming an increase in the antibody titer by the double immunodiffusion, the cervical vein was incised and the whole blood was collected one week after the final sensitization. The blood was allowed to stand at room temperature for 1 hour and then at 4 °C overnight. After centrifuging at 2,000 rpm for 5 minutes, 65 ml of the serum thus obtained was inactivated by heating at 56 °C for 30 minutes. To 50 ml of this serum was added 50 ml of a 34 % (w/v) Na₂SO₄ solution and the precipitate thus formed was centrifuged at 10,000 rpm for 30 minutes. The resulting precipitate was washed with a 17 % (w/v) Na₂SO₄ solution twice and the precipitate was dissolved in 50 ml of 0.1 M Tris-HCl buffer (pH 8.0). 7.5 g of solid Na₂SO₄ was dissolved in this solution under stirring. The precipitate thus formed was dissolved in the same Tris-HCl buffer as used above. Further, the precipitation procedure was repeated thrice by using Na₂SO₄ in a concentration of 7.5 g/50 ml and the precipitate thus finally obtained was dissolved in the above-mentioned buffer. Subsequently, it was desalted by passing through a Cellulofine GH-20m column (2.8 x 90 cm, eluted with 0.05 M NH₄HCO₃) equilibrated with 0.05 M NH₄HCO₃. The elute was freeze-dried to obtain an IgG fraction of anti-factor G serum.

### Example 3

### Production of monoclonal antibody to purified factor G

0.5 ml (200 µg protein/ml) of factor G obtained in Example 2 was mixed with the equivalent amount of Freund's complete adjuvant. Then, the resulting mixture was subcutaneously injected into the back (0.2 ml) and the hip (0.3 ml) of a mouse (BALB/c, aged 5 weeks, 25 g body weight). The second sensitization was made two weeks after the first sensitization and the final immunization was made by intravenously administering 0.3 ml of 300 µg/ml factor G three weeks after the first sensitization. Four days thereafter, 9.2 x 10⁷ spleen cells were separated and allowed to fuse with 1.8 x 10⁷ mouse myeloma SP/0 cells in a conventional manner to thereby give hybridomas. It was confirmed that the resulting hybridomas would bind to factor G and neutralize factor G activity. Next, 0.2 ml of pristan (2,6,10,14-tetramethylpentadecane) was intraperitoneally administered to a similar mouse as used above. After 1 week, 3 x 10⁷ cells/mouse of the hybridomas were intraperitoneally administered to the mouse. In the second week, a large amount of ascites was pooled. The ascites was then collected and the IgG fraction was precipitated with 40 %-saturated ammonium sulfate. Thus, an ascitic form monoclonal antibody was finally obtained.

### Example 4

### Preparation of factor G-free lysate by using anti-factor G antibody-immobilized Cellulofine

100 ml of the lysate obtained by the method described in Example 1 was applied to an endotoxin and β-glucan-free anti-factor G antibody-immobilized Cellulofine column (1.2 x 11 cm), which had been equilibrated with a 0.1 M Tris-HCl buffer (pH 8.0, containing 0.15 M NaCl), prepared by a method as described below and washed with a 0.1 M Tris-HCl buffer (pH 8.0, containing 1 M NaCl). Then the nonadsorbed fractions passed through the column were collected to obtain a factor G-free lysate containing substantially no factor G.

### [Preparation of anti-factor G antibody-immobilized Cellulofine]

10 g of formylcellulofine was sufficiently washed with a 0.1 M sodium phosphate buffer (pH 7.1) and suspended in 20 ml of a solution of each antibody to the respective factors G obtained in Examples 1 to 3 (10 mg/ml 0.1 M sodium phosphate buffer, pH 7.1). Then, 50 mg of NaCNBH₃ was added thereto to obtain a suspension. After slowly stirring at room temperature for 8 hours, it was washed with a 0.2 M Tris-HCl buffer (pH 7.0) and filtered. Subsequently, 5 ml of the above-mentioned buffer containing 10 mg of NaCNBH₃ was added thereto followed by shaking at room temperature for 3 hours. Then, it was sufficiently washed with a 0.1 M Tris-HCl buffer (pH 8.0, containing 0.15 M NaCl).

### Example 5

### Assay of endotoxin

Three reagents were produced by the following methods and their reactivities with 3 samples were examined for comparison.

The reagent A was produced by mixing 440 µl of the lysate, 440 µmol of magnesium chloride and 2.86 µmol of Boc-Leu-Gly-Arg-pNA followed by freeze-drying. The agent B was produced by adding 180 µl of a 10 mg/ml solution of the IgG fraction of the anti-factor G fraction serum produced in Example 1 in a 0.02 M Tris-HCl buffer (pH 8.0) to the components of the reagent A followed by freeze-drying. The reagent C was produced by adding 180 µl of a 10 mg/ml solution of the IgG fraction of the anti-factor G serum produced in Example 2 in a 0.02 M Tris-HCl buffer (pH 8.0) to the components of the reagent A followed by freeze-drying.

These three reagents were each dissolved in 2.2 ml of a 0.2 M Tris-HCl buffer (pH 8.0) and 0.1 ml portions of the resulting solution were pipetted into test tubes. 0.1 ml of a sample was added thereto and mixed well. The mixture was allowed to react at 37 °C for 30 minutes. The reactivities of the three reagents with the samples were examined by inducing the coloration of the pNA formed after 30 minutes by successively adding 0.5 ml of 0.04 % sodium nitrite (in 0.48 M hydrochloric acid), 0.3 % ammonium sulfamate and 0.07 % N-(1- naphthyl)ethylenediamine dihydrochloride and measuring the absorbance at 545 nm. Table 1 shows the results. As these results clearly show, the endotoxin can be specifically assayed by using a reagent, which includes a polyclonal antibody to the factor G fraction and a polyclonal antibody to factor G, without being affected by (1 → 3)-β-D-glucan.

**Table 1**

| Sample (pg/tube) | | Reactivity (ΔA545 nm/30 min) | | |
|---|---|---|---|---|
| Endotoxin* | Glucan** | Reagent A no antibody | Reagent B factor G fraction antibody | Reagent C factor G antibody |
| | 3.0 | 0.242 | 0.001 | 0.001 |
| 2.5 | | 0.447 | 0.445 | 0.448 |
| 2.5 | 3.0 | 0.691 | 0.447 | 0.446 |

| | | | | |
|---|---|---|---|---|
| *: Derived from E. coli 0111:B4. | | | | |
| **: Curdlan. | | | | |

### Example 6

### Assay of endotoxin

Two reagents were produced by the following methods and their reactivities with an endotoxin and (1 → 3)-β-D-glucan were and examined for comparison.

The reagent A was produced by mixing 440 µl of the lysate, 440 µmol of magnesium chloride and 2.86 µmol of Boc-Leu-Gly-Arg-pNA followed by freeze-drying. The reagent D was produced by adding 80 µl of a solution containing the monoclonal antibody capable of neutralizing the purified factor G prepared in Example 3 to the components of the reagent A followed by freeze-drying.

These two reagents were each dissolved in 2.2 ml of a 0.2 M Tris-HCl buffer (pH 8.0) and 0.1 ml portions of the resulting solution were pipetted into test tubes. 0.1 ml of a sample was added thereto and mixed well. The mixture was allowed to react at 37 °C for 30 minutes. The reactivities of the two reagents with the samples were examined by inducing the coloration of the pNA formed after 30 minutes by successively adding 0.5 ml of 0.04 % sodium nitrite (in 0.48 M hydrochloric acid), 0.3 % ammonium sulfamate and 0.07 % N-(1-naphthyl)ethylenediamine dihydrochloride and measuring the absorbance at 545 nm.

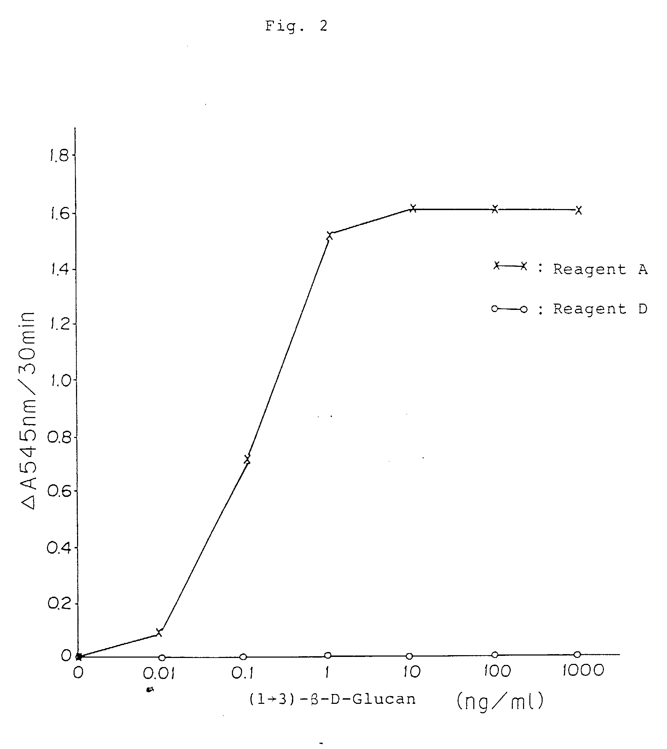

Fig. 2 shows the results of the comparison of the reactivity of the reagents with (1 → 3)-β-D-glucan. The reagent A reacted with the (1 → 3)-β-D-glucan depending on concentration, while the reagent D never reacted with (1 → 3)-β-D-glucan of 1,000 ng/ml. These results indicate that the monoclonal antibody to the purified factor G had completely neutralized factor G in the lysate and thus its reactivity with (1 → 3)-β-D-glucan had been eliminated.

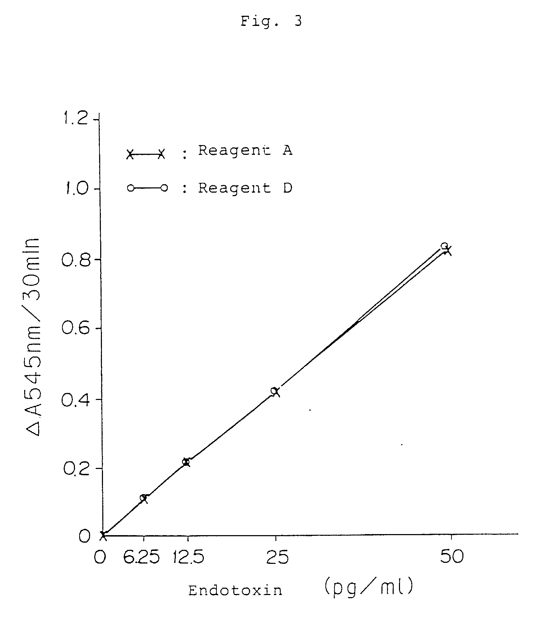

Fig. 3 shows the comparison of the reactivities of the reagents A and D with the endotoxin with the use of the dose-response curves of these reagents. As Fig. 2 shows, the dose-response curves of these reagents almost agree with each other. This fact means that the monoclonal antibody to factor G in the reagent D never affected the reactivity of the lysate with endotoxin.

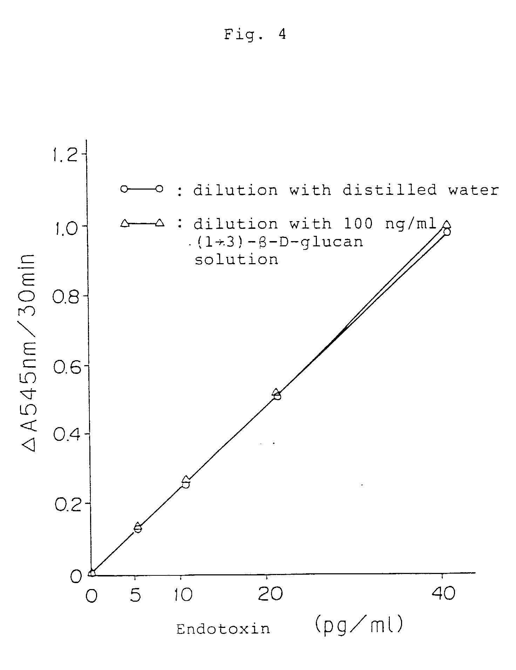

Fig. 4 shows the dose-response curves of the reagent D against a serial dilution of the endotoxin with distilled water and another serial dilution thereof with a 100 ng/ml (1 → 3)-β-D-glucan solution. These two dose-response curves almost agree with each other, which means that the endotoxin can be specifically assayed, without being affected by (1 → 3)-β-D-glucan contained in a sample, by using the agent D.

These results clearly indicate that an endotoxin can be specifically assayed, without being affected by (1 → 3)-β-D- glucan, by using a reagent including a monoclonal antibody to the purified factor G.

### Example 7

### Assay of endotoxin

Two reagents were produced by the following methods and their reactivities with 3 samples were examined for comparison.

The reagent A was produced by mixing 440 µl of the lysate, 440 µmol of magnesium chloride and 2.86 mol of Boc-Leu-Gly-Arg-pNA followed by freeze-drying. The reagent E was produced by mixing 440 µl of the factor G-free lysate produced in Example 4 with 2.86 µmol of Boc-Leu-Gly-Arg-pNA, followed by freeze-drying.

These two reagents were each dissolved in 2.2 ml of a 0.2 M Tris-HCl buffer (pH 8.0) and 0.1 ml portions of the resulting solution were pipetted into test tubes. 0.1 ml of a sample was added thereto and mixed well. The mixture was allowed to react at 37 °C for 30 minutes. The reactivities of the two reagents with the samples were examined by inducing coloration of the pNA formed after 30 minutes by successively adding 0.5 ml of 0.04 % sodium nitrite (in 0.48 M hydrochloric acid), 0.3 % ammonium sulfamate and 0.07 % N-(l-naphthyl)ethylenediamine dihydrochloride and measuring the absorbance at 545 nm. Table 2 summarizes the results. As these results clearly show, the endotoxin can be specifically assayed by using a reagent, which has been produced by using the aforesaid factor G-free lysate, without being affected by (1 → 3)-β-D-glucan.

**Table 2**

| Sample (pg/tube) | | Reactivity (ΔA545 nm/30 min) | |
|---|---|---|---|
| Endotoxin* | Glucan** | Reagent A lysate | Reagent E factor G-free lysate |
| | 3.0 | 0.227 | 0.001 |
| 2.5 | | 0.439 | 0.437 |
| 2.5 | 3.0 | 0.668 | 0.439 |

| | | | |
|---|---|---|---|
| *: Derived from E. coli 0111:B4. | | | |
| **: Curdlan. | | | |

### Example 8

### Assay of plasma specimen

Subjects were 25 patients being in the hospital of the Department of Hematology attached to Jichi Medical School, who suffered from hepatic and bile duct diseases accompanied by serious hemopathy such as leukemia and infection and further seemed to suffer from septicaemia due to gram-negative bacteria. Blood was aseptically collected from each subject followed by adding heparin to serve as a sample. The heparin-added blood samples were centrifuged at 4 °C at 150 x G for 10 minutes to give platelet rich plasma (PRP) samples. To 0.1 ml of each PRP sample was added 0.2 ml of 0.32 M perchloric acid and the mixture was incubated at 37 °C for 20 minutes. The precipitate was removed by centrifugation at 3,000 rpm for 10 minutes. 0.05 ml of the resulting supernatant was neutralized with 0.05 ml of 0.18 M NaOH. Thus a specimen was obtained.

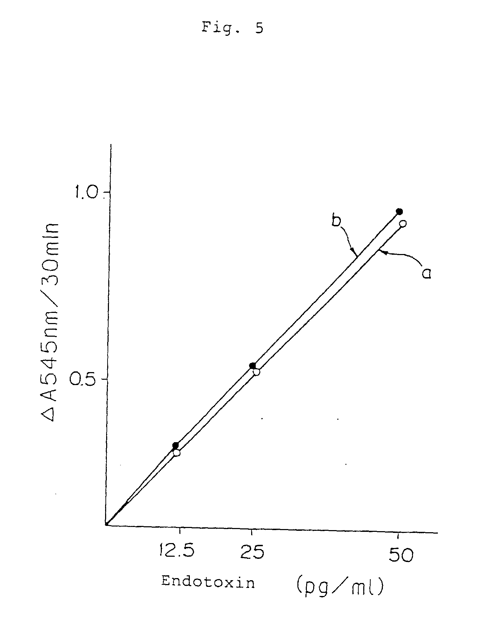

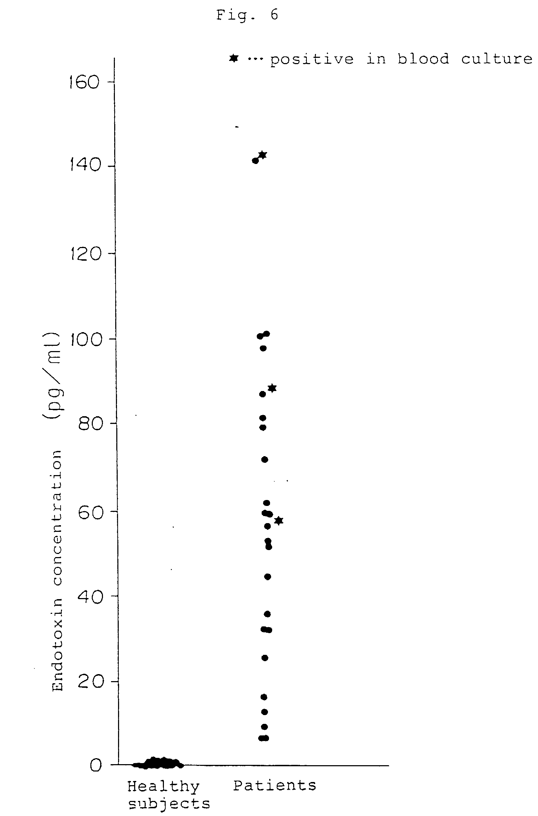

Next, 0.1 ml of the endotoxin assay reagent of the present invention produced by the method described in Example 6 was added thereto and the mixture was incubated at 37 °C for 30 minutes. To the resulting solution, 0.5 ml portions of 0.04 % sodium nitrite (in 0.48 M hydrochloric acid), 0.3 % ammonium sulfamate and 0.07 % N-(1-naphthyl)ethylenediamine dihydrochloride were successively added to thereby perform diazo-coupling. Then, the absorbance of the resulting mixture was measured at 545 nm. The level of the E. coli 0111:B4 endotoxin was calculated from the calibration curve a (Fig. 5) which had been separately prepared. As Fig. 6 shows, the endotoxin was detected at high concentrations in all of the 25 samples (healthy 25 subjects: 0.8 ± 0.6 pg/ml). Further, Escherichia coli, Pseudomonas aeruginosa and Klebsiella pneumoniae were respectively detected from 3 (*) out of 25 samples by blood culture. Although the remaining 22 samples showed negative results in blood culture, they were diagnosed as gram-negative bacterial septicaemia based on clinical symptoms including fever and leukocyte count as well as sensitivities for antibiotics. Thus, it can be understood that the method of the present invention is highly useful in rapidly diagnosing gram-negative bacterial septicaemia which can be hardly diagnosed by known test methods.

### Example 9

### Assay of urine specimen

The endotoxin was assayed according to the method of the present invention for the urine of 3 patients suffering from complicated urinary tract infection while being in the hospital attached to Jichi Medical School, in which Escherichia coli and Serratia marcescens had been detected by urine culture.

The intermediate urine of each subject was aseptically collected in a sterilized cup. To 0.005 ml of this urine was added 0.2 ml of the endotoxin assay reagent of the present invention produced by the method described in Example 7 and the resulting mixture was incubated at 37 °C for 30 minutes. After performing diazo-coupling in the same manner as in Example 8, the absorbance of the solution was determined at 545 nm. The level of E. coli 0111:B4 endotoxin was calculated from the calibration curve b (Fig. 5) which had been separately prepared. As Table 3 shows, the endotoxin was detected at high concentrations in all of the samples (healthy subjects: 60 pg/ml or less). Thus, it can be understood that the method of the present invention is highly useful in rapidly diagnosing gram-negative bacterial urinary tract infection.

**Table 3**

| Endotoxin concentration in urine of subjects suffering from gram-negative bacterial infection | | | |
|---|---|---|---|
| No. | Detected bacterium | CFU* /ml | Endotoxin (ng/ml) |
| 1 | Escherichia coli | > 10⁵ | 1056.5 |
| 2 | Serratia marcescens | > 10³ | 18.0 |
| 3 | Serratia marcescens | > 10⁴ | 216.7 |

| | | | |
|---|---|---|---|
| *: Colony-forming unit. | | | |

### Example 10

### Assay of cerebrospinal fluid specimen

The endotoxin was determined by the method of the present invention for cerebrospinal fluid samples collected from 3 subjects who had seemed to suffer from meningitis in the hospital attached to Jichi Medical School, from which Pseudomonas aeruqinosa and Haemophilus influenzae had been detected.

0.05 ml of distilled water for injection was added to 0.05 ml of the cerebrospinal fluid of each subject aseptically collected by lumbar puncture. Further, 0.1 ml of the endotoxin assay reagent of the present invention produced by the method described in Example 5 was added thereto and the resulting mixture was incubated at 37 °C for 30 minutes. After performing diazo-coupling in the same manner as in Example 8, the absorbance of the solution was determined at 545 nm. The level of the E. coli 0111:B4 endotoxin was calculated from the calibration curve b (Fig. 5) which had been separately prepared. As Table 4 shows, the endotoxin was detected at high concentrations in all of the 3 samples (healthy subjects: 3 pg/ml or less). Thus, it can be understood that the method of the present invention is highly useful in rapidly diagnosing gram-negative bacterial meningitis in the early stage.

**Table 4**

| Endotoxin concentration in cerebrospinal fluid of subject suffering from gram-negative bacterial infection | | |
|---|---|---|
| No. | Detected bacterium | Endotoxin (pg/ml) |
| 1 | Pseudomonas aeruginosa | 75.5 |
| 2 | Pseudomonas aeruginosa | 108.5 |
| 3 | Haemophilus influenzae | 34.6 |

### INDUSTRIAL APPLICABILITY

As described above, the present invention provides a reagent comprising the lysate for specifically assaying an endotoxin whereby an endotoxin originating from gram-negative bacteria contained in biological samples (for example, blood, urine, cerebrospinal fluid) can be rapidly and easily assayed at a high accuracy. Therefore, the assay reagent of the present invention is useful in rapidly diagnosing gram-negative bacterial septicaemia and endotoxemia and evaluating therapeutic effects on these diseases. Thus, it greatly contributes to, in particular, clinical diagnosis.

Furthermore, the present invention makes it possible to rapidly and accurately assay an endotoxin contaminating distilled water for injection, medical devices and injections, such a secondary effect according to the present invention greatly contributes to the field of the drug manufacturing industries.

## Claims

1. A reagent for assaying an endotoxin which contains limulus amoebocyte lysate, an antibody to (1→3)-β-D-glucan sensitive factor and a synthetic peptide substrate having an amino acid sequence comprising a hydrolysis site of coagulogen.

2. A reagent for assaying an endotoxin as claimed in claim 1, wherein said antibody is a monoclonal antibody.

3. A reagent for assaying an endotoxin as claimed in claim 1, wherein said antibody is a polyclonal antibody.

4. A method of assaying an endotoxin in a sample using a limulus amoebocyte lysate which comprises the steps of:
(a) combining a solution of the limulus amoebocyte lysate with a solution of an antibody to a (1→3)-β-D-glucan sensitive factor to eliminate any effects of the (1→3)-β-D-glucan sensitive factor in the lysate;
(b) combining the lysate solution obtained in (a) with a sample to cause stepwise activation of coagulation factors in the lysate which comprises activating an endotoxin sensitive factor in the lysate by reacting it with an endotoxin in the sample, activating factor B with the active endotoxin sensitive factor and converting proclotting enzyme to clotting enzyme with the active factor B, and reacting the clotting enzyme with a synthetic peptide substrate having an amino acid sequence comprising a hydrolysis site of coagulogen; and
(c) measuring a change of coagulogen or the synthetic peptide substrate.

## Patentansprüche

1. Reagens zum Test eines Endotoxins, welches Limulus-Amoebocyten-Lysat, einen Antikörper gegen (1 → 3)-β-D-Glucan-empfindlichen Faktor und ein synthetisches Peptidsubstrat mit einer Aminosäuresequenz entsprechend einer Hydrolysestelle von Coagulogen enthält.

2. Reagens zum Test auf Endotoxin nach Anspruch 1, worin der Antikörper ein monoklonaler Antikörper ist.

3. Reagens zum Test auf Endotoxin nach Anspruch 1, worin der Antikörper ein polyklonaler Antikörper ist.

4. Verfahren zum Testen eines Endotoxins in einer Probe unter Verwendung von Limulus-Amoebocyten-Lysat, welches die Schritte umfaßt:
(a) Zusammengeben einer Lösung des Limulus-Amoebocyten-Lysats mit einer Lösung eines Antikörpers gegen (1 → 3)-β-D-Glucan-empfindlichen Faktor unter Eliminierung alle Effekte des gegen (1 → 3)-β-D-Glucan-empfindlichen Faktors im Lysat;
(b) Zusammengeben der in (a) erhaltenen Lysatlösung mit einer Probe unter Bewirkung einer schrittweisen Aktivierung der Koagulationsfaktoren im Lysat, welches das Aktivieren eines Endotoxinempfindlichen Faktors im Lysat durch Reaktion mit einem Endotoxin in einer Probe umfaßt, Aktivieren von Faktor B mit aktivem Endotoxin-empfindlichem Faktor und Umwandeln von Pro-Gerinnungsenzym in Gerinnungsenzym mit aktivem Faktor B und Umgeben des Gerinnungsenzyms mit einem synthetischen Peptidsubstrat mit einer Aminosäuresequenz, die eine Hydrolysestelle des Coagulogens umfaßt; und
(c) Messen der Veränderung am Coagulogen oder synthetischen Peptidsubstrat.

## Revendications

1. Réactif de dosage d'une endotoxine, lequel contient du lysat d'amibocytes de limule, un anticorps dirigé contre le facteur sensible au (1 → 3)-β-D-glycane et un substrat peptidique de synthèse ayant une séquence d'acides aminés comprenant un site d'hydrolyse du coagulogéne.

2. Réactif de dosage d'une endotoxine selon la revendication 1, où ledit anticorps est un anticorps monoclonal.

3. Réactif de dosage d'une endotoxine selon la revendication 1, où ledit anticorps est un anticorps polyclonal.

4. Procédé de dosage d'une endotoxine dans un échantillon par utilisation d'un lysat d'amibocytes de limule, lequel comprend les étapes consistant à :
(a) combiner une solution du lysat d'amibocytes de limule avec une solution d'un anticorps dirigé contre un facteur sensible au (1 → 3)-β-D-glycane, pour éliminer tout effet du facteur sensible au (1 → 3)-β-D-glycane dans le lysat ;
(b) combiner la solution de lysat obtenue en (a) avec un échantillon pour provoquer l'activation graduelle des facteurs de la coagulation dans le lysat, laquelle comprend l'activation d'un facteur sensible à l'endotoxine dans le lysat par mise à réagir avec une endotoxine dans l'échantillon, l'activation du facteur B avec le facteur sensible à l'endotoxine actif et la conversion de l'enzyme de procoagulation en enzyme de coagulation par le facteur B actif, et la mise à réagir de l'enzyme de coagulation avec un substrat peptidique de synthèse ayant une séquence d'acides aminés comprenant un site d'hydrolyse du coagulogène ; et
(c) mesurer la modification du coagulogène ou du substrat peptidique de synthèse.
